# EUROPEAN PATENT APPLICATION

(11) **EP 1 800 696 A1**
(43) Date of publication of application: **27.06.2007**
(21) Application number: 05112966.6
(22) Date of filing: 23.12.2005
(51) Int. Cl.: A61K 47/48

(54) **MBP-mediated molecular cargo delivery into cells**

(71) Applicant: Universita' Degli Studi di Torino, 10126 Torino (IT)
(72) Inventor: Accornero, Federica, 14030, VIARIGI (ASTI) (IT); Brancaccio, Mara, 11027, SAINT VINCENT (AOSTA) (IT); Silengo, Lorenzo, 10134, TORINO (IT); Tarone, Guido, 10131, TORINO (IT); Turco, Emilia, 10126, TORINO (IT)
(74) Representative: Freyria Fava, Cristina

(57) **Abstract**

The present invention concerns the delivery of a molecule of interest (cargo moiety) into cells using a novel membrane translocating protein domain fused and/or coupled to said molecule of interest. In particular, the present invention concerns a system and a method for the delivery a molecule of interest into cells by means of fusion to maltose binding protein.

## Description

### Field of the Invention

The present invention concerns the delivery of compounds into cells using a novel membrane translocating protein domain fused and/or coupled to the compounds of interest.

### Background of the invention.

Delivery of molecules inside the cell is of great interest both for research and pharmacological purposes. Entry of hydrophilic molecules is strictly controlled by the cells and most molecules of potential pharmacological interest, such as proteins and nucleic acids, do not enter the cell due to the impermeability of the cell membrane. A significant limitation for cellular research, and in the development of many therapeutic drugs is, thus, the poor permeability of the cell membrane to outside agents. What is needed is a method for importing entire protein molecules into a cell for studies of intracellular processes in living systems, for drug delivery, for vaccine development, and for disease therapy.

Several approaches have been tried, but all have limitations. One approach has made use of synthetic vectors derived from lipids such as liposomes (see, e. g., Leserman et al. (1980) Nature (London) 288,602;Machy and Leserman, (1983), Biochim. Biophys. Acta, 730,313), cationic lipids (see, e.g., Felgner et al., (1987) Proc. Natl. Acad. Sci. USA, 84, 7413; Behr et al.(1989) Proc. Natl. Acad. Sci. USA, 86,6982; Demeneix et al. (1991) Int. J. Dev.Biol., 35,481; Labat-Moleur et al. (1996), Gene Ther., 3,1010; Zhou et al. (1994), Biochim. Biophys. Acta, 1189, 195; Radler et al (1997), Science, 275,810) and polyethyleleimine (see, e. g.,Boussif et al. (1996), Gene Ther., 3,1074;Boletta etal., (1997), Hum. Gene Ther., 8,1243). These, while demonstrated to be somewhat effective for the delivery of nucleic acids, have largely not proven successful delivering other types and sizes of compounds. Moreover, they represent foreign entities that will likely cause side effects and cellular toxicity.

Another approach has been the use of viral vectors. Although a powerful technology unto itself, this type of vector can only directly introduce nucleic acids, not proteins. Furthermore, these types of vector present difficulties, or at least apprehension, and hence reluctance, based on concerns of mutagenicity as well as antigenicity. So far, these safety concerns have yet to be adequately resolved.

Still another approach has been the use of microinjection, but this mechanical procedure is time consuming and not practical from the standpoint of delivery to whole populations of cells, and cells that may already be differentiated, or else in situ deep inside a patient.

More recently, vectors have been described that are based on peptides that have membrane translocation capacity. These include polylysine (see, e. g., Leonetti et al. (1988), Gene, 72,323 ; Degols et al. (1989) Nucleic Acids Res., 19,945;Lemaitre et al. (1987), Proc. Natl. Acad. Sci. USA, 84,648; and Degols et al. (1994), Bioconjug. Chem., 5,8), fusion peptides (see, e.g., Pichon et al. (1997), Mol. Pharmacol., 51,431; andBongartz et al. (1994), Nucleic AcidsRes., 22,, 4681), peptides issued from the homeodomain of antennapedia (see, e. g., Derossi et al., (1996), J. Biol. Chem., 271,18188; and Brugidou et al. (1995), Biochem. Biophys. Res. Commun., 214,685) and short peptides such as KDEL (see Arar et al. (1995), Bioconjug. Chem., 6,573), sequences related to the tat gene of HIV (Vives et al. (1997), J. Biol. Chem., 272,16010), and more sophisticated "loligomer" peptides that contain a nuclear localization sequence associated with an oligolysine sequence (see Sheldon et al. (1995), Proc. Natl., Acad. Sci. USA, 92,2056. Another vector has further made use of basic peptides (see, e. g., Niidome et al., H., 1997, J. Biol. Cham., 272,15307; Haensler et al. (1993), Bioconjugate Chem., 4,372 ; and Gottschalk et al. (1996), Gene Therapy, 3,448). Finally, a short peptide has previously been shown to effectively deliver small oligonucleotides of 18-36 nucleotides in length when non-covalently associated therewith (Morris et al. (1997) Nucleic Acids Res., 25,2730). Most, if not all, of these existing techniques are limited by a lack of delivery efficiency, especially for large macromolecules, and further yet from compromised biological activity due to serum or medium sensitivity and cytotoxicity. A second limitation is due to the difficulty of purification of the cargo and/or the vector/cargo complex before administration to organisms since all the peptide-based vectors system developed up to now require the addition of tags sequences suitable for such purification purpose.

The present invention overcome both of the above noted deficiencies in the art as it allows good delivery efficiency for large hydrophilic molecules and a broad spectrum of compounds in different cell types. In addition it allows a rapid, simple and cost effective purification of the vector and/or the vectorcargo fusion protein.

### Summary of the Invention

Object of the present invention is a method and a system for the delivery of compounds into cells.

The method and system according to the present invention can be used in a variety of applications, including, but not limited to, studies of intracellular protein function, vaccine delivery, and delivery of proteins, peptides, nucleic acids, and other organic compounds for therapeutic use.

According to the present invention, said objects are achieved thanks to the solution having the characteristics referred to specifically in the ensuing claims. The claims form integral part of the technical teaching herein provided in relation to the present invention.

The present invention relates to the ability of the maltose binding protein, either recombinantly or naturally produced, to translocate across the cell membrane and to import into cells biologically active cargo molecules, such as proteins, nucleic acids organic compounds, drugs or vaccines associated with it.

### Brief description of the drawings

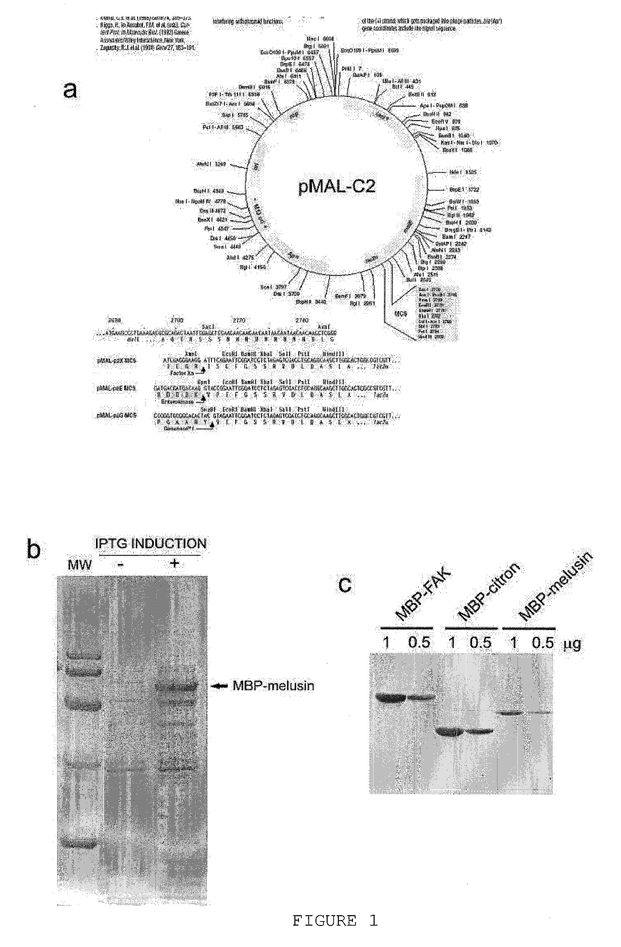

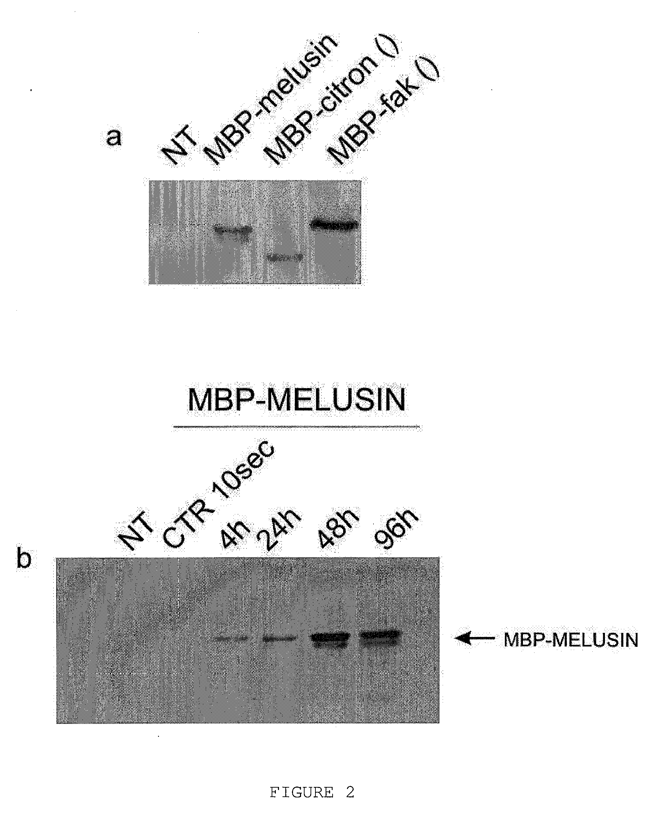

- Figure 1: Recombinant protein preparation.
   a) pMal-C2 plasmid map used to clone human melusin, mouse citron kinase fragment (coding for aa 711-894) and chicken FAK fragment (coding for aa 700-1033) cDNAs. b) Protein extracts from E. coli transformed with expression plasmid coding for melusin either before and after induction with IPTG were analyzed with SDS page electrophoresis followed by coomassie blue staining. Note the presence of the recombinant protein in the induced cell extract (arrow). MW: molecular weight marker c) coomassie blue staining of MBP-melusin, MBP-citron and MBP-FAK fusion proteins after affinity purification on amylose-sepharose column from bacterial cell extracts
- Figure 2: MBP-fusion protein uptake by in vitro cultured cells.
   a) COS cells untreated (NT) or treated for 24 hours with 50 µg/ml of recombinant MBP-melusin, MBP-citron and MBP-FAK were washed 10 times with 10 ml of PBS and extracted with lysis buffer (60 mM Tris pH 6.8, 1 % SDS). Total cell extracts were subjected to SDS-PAGE and western blot analysis with anti-MBP polyclonal antibody.
   b) Primary rat cardiomyocytes untreated (NT) or treated for different time (10 seconds, 4, 24, 48, 96 hours) with 50 µg/ml of recombinant MBP-melusin were washed 10 times with 10 ml of PBS and extracted with lysis buffer (60 mM Tris pH 6.8, 1 % SDS). Total cell extracts were subjected to SDS-PAGE and western blot analysis with anti-melusin polyclonal antibody.

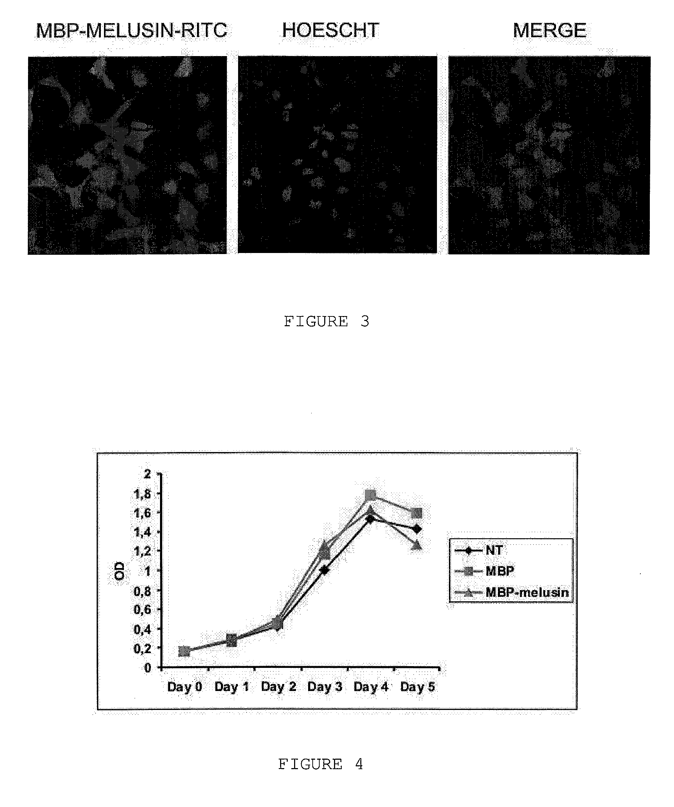

- Figure 3: Subcellular localization of MBP-melusin. COS cells were incubated for 2 hours with 50 µg/ml of recombinant TRITC-conjugated MBP-melusin and analysed with confocal microscopy (a). Nuclei were stained with hoescht dye (b). The two images in a and b are superimposed (c). Note the uniform distribution of melusin in cytoplasm and nucleus.
- Figure 4: MBP and MBP-melusin are not toxic for the cells. 50.000 COS cells/well were plated in 6 well dishes. Cells were grown in standard culture medium (NT) or in presence of 50 µg/ml of recombinant MBP or MBP-melusin for different times (0, 1, 2, 3, 4, 5 days). Cell density was evaluated by crystal violet staining. After repeated washing, the dye was eluted and quantified by spectrophotometric analysis. Optic density (OD) at 590 nm were reported in the graph. No effect of MBP or MBP-melusin can be detected on cell over a period of 5 days.

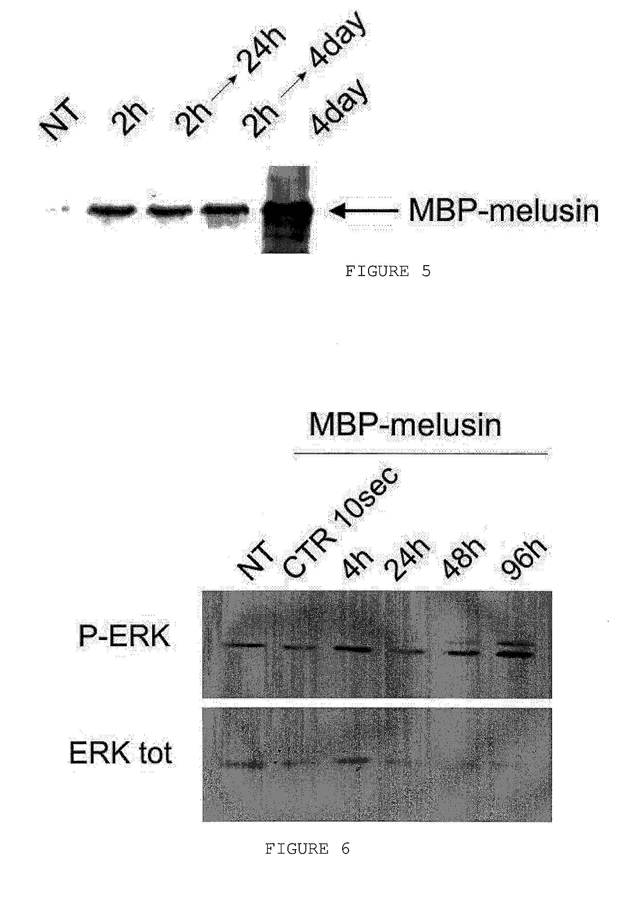

- Figure 5: Stability of MBP-melusin inside the cells. COS cells were incubated with 50 µg/ml of recombinant MBP-melusin for 2 hours and then lysed immediately, after 24 hours or 4 days. As control cells were treated for 4 days and lysed immediately. Total cell extracts from the different samples and from untreated COS cells (NT) were subjected to western blot analysis using a anti-melusin antibody. Note that the intracellular level of MBP-melusin remain steady state for 24 hours and even 4 days.
- Figure 6: MBP-melusin is biologically active within the cells. Primary rat cardiomyocytes untreated (NT) or treated for different time (10 seconds, 4, 24, 48, 96 hours) with 50 µg/ml of recombinant MBP-melusin were washed 10 times with 10 ml of PBS and extracted with lysis buffer (60 mM Tris pH 6.8, 1 % SDS). Total cell extracts were subjected to western blot analysis with anti-phospho ERK1/2 (upper panel) and anti-total ERK1 (lower panel) polyclonal antibodies. Note that the amount of phospho ERK1/2 is increased in cells treated for 48 and 96 hours indicating that intracellular melusin can trigger activation of this kinase.

### Detailed description of the invention

The present invention will now be described in detail in relation to some preferred embodiments by way of non-limiting examples.

The present invention is based on the discovery that the maltose binding protein (MBP), produced as recombinant protein i.a. in *E. Coli* and subsequently purified by affinity chromatography, is capable to translocate across the cell membrane and enter the cytoplasm of cells grown in vitro when incubated in the culture medium.

When the MBP coding sequence is fused to DNA sequence coding for a protein of interest the resulting recombinant protein retains the ability to translocate across the cell membrane and enter the cytoplasm, thus providing an efficient and effective delivery of the molecule of interest into the cell.

The molecules fused to MBP can be of different size and nature. As an example a protein capable to bind a specific ligand of interest can be fused to MBP allowing to deliver such ligand inside the cell. Ligand molecules can be proteins, nucleic acids or organic compounds.

As an alternative method to the fusion protein generated by recombinant DNA technology, MBP can be linked to the cargo molecule of interest by chemical means. The purified MBP can be covalently cross-linked to a purified molecule by standard in vitro reactions. This will allow to generate an MBP/cargo complex useful to deliver into the cells peptides, proteins as well as compounds of different chemical nature.

As an alternative to the method described above the cargo molecule of interest can be coupled to maltose by chemical means and subsequently bound to purified MBP exploiting its maltose binding activity. This allows to generate a non covalent MBP-cargo complex that upon delivery to cells allows to separate the active cargo from the MBP carrier. Cargo molecules can include proteins, peptides, nucleic acids and organic compounds.

The method of the present invention provides a means for producing organic molecules capable to penetrate into the interior of the cell, for a variety of research studies on mechanisms of intracellular metabolism, receptor signalling, apoptosi, cell cycle control.

This method also provides a means to introduce molecules into cells for pharmacological treatment of a variety of human and animals pathologies, such as cancer, inflammation, infectious diseases, cardiovascular disorders, heart and kidney failure, diabetes.

With respect to the cancer therapy, the method of the present invention provides a means for producing cell-permeable molecules (e.g. proteins) for the treatment of cancer. Regulators of apoptosis and cell cycle control have been found to play a key role in oncogenesis, and gene therapy techniques using intratumoral injection of an adenoviral expression vector encoding the p53 gene have shown promise for the control of some tumors. Delivery of specific protein products through the use of viral vectors has proven to be problematic, however. The method of the present invention provide a means for producing cell-permeable proteins from among the cell cycle regulators and regulators of apoptosis, as well as other proteins identified to play a role in the development of the cancer state. Administration of cell-permeable protein can be accomplished in various ways, including, but not limited to, intratumoral injection, infusion, and intravenous administration. Bax and Bcl-xL are other examples from among a wide variety of proteins that have been determined to effect cell cycle control and apoptosis, and therefore be effective for cancer therapy. The method of the present invention provides a more efficient, less labor-intensive, less costly method for delivery of anti-oncogenic proteins to tumor cells.

With respect to cardiac and vascular pathologies, the method of the present invention provides a means for producing cell-permeable molecules capable to penetrate the cells and activate compensatory heart hypertrophy useful to treat and prevent cardiac dilation and failure.

Melusin (Brancaccio et al. 1999; GenBank AF140690; GenBank AF140691) is a protein specifically present within cardiomyocytes (the cells developing contractile power) and acts as a biochemical switch triggering cardiomyocyte hypertrophy (and thus increased contractile power). A melusin-based therapy represents thus an entirely novel strategy based on the ability of this protein to act on cardiomyocyte and stimulate the beneficial hypertrophic response necessary to increase heart contractile power. Since melusin is an intracellular protein a recombinant molecule consisting of MBP-melusin fusion can be an ideal solution. MBP-melusin can be administrated by intra-coronary injections to specifically target the myocardium in experimental animal models of heart failure. An alternative method of administration will be by intra-myocardial injections in the left ventricle walls. Hypertension is a major pathology affecting million of peoples and availability of cell permeable proteins, peptides or nucleotides will allow to target the intracellular molecules involved in the control of vascular tone.

The development of a means of enhancing topical absorption of drugs is thus urgently needed to treat pathologies such as psoriasis and atopic dermatitis that require topical pharmacological treatment since systemic administration can lead to considerable side effects.

Psoriasis affects approximately 2% of the population in Western countries and is one of the most common inflammatory skin disorders. Recently it has been shown that Stat3 is activated in keratinocytes of human psoriatic lesions. Furthermore, transgenic mice expressing a constitutively active form of Stat3 in keratinocytes developed skin lesions either spontaneously or in response to wounding that closely resembled human psoriasis. Further analyses showed a dual requirement for both activated Stat3 in keratinocytes as well as activated T lymphocytes in skin of transgenic mice for development of psoriatic lesions. Inhibition of Stat3 function by intracellular decoy oligonucleotides inhibits the onset and reverses established psoriatic lesions in mice expressing activated Stat3 in keratinocytes. Thus, targeting Stat3 with intracellular inhibitors such as dominant negative mutant proteins, or with short decoy peptide sequences, or with siRNA molecules, can represent potential therapeutic approaches in the treatment of psoriasis (Sano et al. 2005).

The present invention can also be employed in the vaccine field. Immunization with a complete protein or protein domain provides a method for introducing epitopes into the cell without having to first isolate the peptides containing them. The antigen-processing machinery of the cell then provides the antigen processing necessary to invoke a protective immune response.

Previously, entire proteins or protein domains could not be delivered to the interior of the cell for processing to occur. As a consequence, peptides representing antigenic epitopes had to be identified prior to delivery to the cell of small peptides representing those epitopes. The method of the present invention allows whole proteins or protein domains to be imported into the cell, where antigenic processing can occur. This provides multiple antigenic epitopes in one administration, and eliminates the need for experimental identification of specific epitopes for vaccine development.

The method of the present invention can also be used to label cells to be tracked in vivo or in vitro, for example, cellular migration through tissue and tumor metastasis. An MBP/GFP protein chimera with specific targeting sequences to efficiently retain the fusion protein within the cell (nuclear targeting sequence) can be administered to cells prior to injection in situ, or can be administered locally in situ, in the case of tumor cells, to study metastasis. Methods for studying cellular migration are known to those of skill in the art.

### EXAMPLES.

### Example 1. Preparation of pMAL constructs and production and purification of recombinant proteins.

The entire human melusin cDNA (ACCESSION BC108901) was cloned in pMal-C2 plasmid (New England Biolabs) (figure 1a) in EcoRI and SalI restriction sites. The mouse cDNA coding for amino acids 711-894 of citron kinase (ACCESSION NM_007708) was cloned in pMal-C2 in a SstI restriction site. The chicken cDNA (ACCESSION M86656) coding for amino acids 700-1033 of FAK was cloned in pMAL-C2 in a PstI restriction site. MBP fusion proteins and MBP alone (ACCESSION J01648) were produced by expressing the pMAL-C2 constructs in IPTG induced Escherichia coli BL21 bacterial strain. In particular, cells were plated onto LB plates containing ampicillin and incubated overnight at 37°C. Colonies were inoculated into LB-Amp culture and grown overnight at 37°C. Overnight cultures were diluted 1:100 with pre-warmed LB-Amp media and grown at 37°C to an OD600 of 0.5-0.6. Protein expression was induced with addition of 0.1 mM isopropylthiogalactoside (IPTG; Sigma, St. Louis, MO) for 4 hours. Cells were centrifuged and resuspended in column buffer (Tris-HCl pH7,4 20mM, NaCl 200mM, EDTA 1uM) containing a protease inhibitor cocktail and then freezed at -80 degrees. Bacterial cells were thaw and sonicated for 6 times for 10 seconds on ice. Cell extract was clarified by centrifugation and the supernatant was transferred in a new tube. Purification of MBP-fusion proteins was performed by affinity chromatography on amylose Sepharose followed by maltose elution according to manufacturer's instructions (New England Biolabs). Protein content in the eluted fractions was determined with Bradford assay and the protein quality was assessed by SDS-PAGE followed by coomassie blue staining (figure 1b).

### Example 2. Treatment of cultured cells with MBP and MBP fusion proteins.

In order to investigate the ability of the recombinant MBP fusion proteins to enter in eukaryotic cells we treated COS cells with 50 µg/ml of recombinant MBP-melusin, MBP-citron and MBP-FAK dissolved in culture medium for 24 hours. Total cell extracts from COS cells untreated and treated with the different MBP fusion proteins were washed 10 times with PBS and then analyzed with SDS-PAGE using an anti-MBP antibody in order to identify the presence of the recombinant proteins in the cells treated. As shown in figure 2a, after 24 hours of treatments MBP-fusion proteins were present in the COS cell extracts, indicating that the recombinant proteins were inside or strictly associated with cells.

A similar experiment was performed on primary rat cardiomyocytes in culture. Cells were treated for different period of time (10 seconds, 4, 24, 48, 96 hours) with 50 µg/ml of recombinant MBP-melusin in the culture medium, then washed 10 times with 10 ml of PBS and extracted. Total cell extracts were subjected to western blot analysis using an anti-melusin antibody. As shown in figure 2b cells as soon as after 4 hours treatment showed the presence of the recombinant melusin in total extracts. The amount of MBP-melusin revealed by western blot increased at 24 hours and picked at 48 hours. After 96 hours of treatments MBP-melusin was still present at a high amount.

### Example 3. Subcellular localization of MBP-fusion proteins

MBP and MBP fusion proteins were conjugated with tetramethylrhodamine isothiocyanate (TRITC, Sigma, USA) in order to follow the subcellular localization of the recombinant proteins after cellular uptake. Figure 5 shows COS cells treated for 2 hours with 50 µg/ml of recombinant TRITC conjugated MBP-melusin and analysed with confocal microscopy. Nuclei were stained with hoescht dye to localize the cells. MBP-melusin is uniformly diffused in the cytoplasm and in the nucleus and, in some cells, seems to be enriched in vesicle-like structures suggesting an endocytosis mediated uptake. Same results were obtained for MBP alone (data not shown).

### Example 4. Cell toxicity assay.

To test the cytotoxicity of the treatment with the MBP-fusion proteins we plated 50.000 COS cells/well in 6 well dishes. Cells were treated with 50 µg/ml of recombinant MBP or MBP-melusin for 0, 1, 2, 3, 4, 5 days and then analyzed for their proliferation ability and compared with untreated cells. Cells were fixed in 4% PFA, stained with crystal violet solution and the dye was recovered and subjected to spectrophotometric quantification. Our results demonstrated that the proliferation curves obtained for COS cells treated with MBP or with MBP-melusin were not significantly different from that obtained for untreated cells (figure 3). This experiment demonstrate that uptake from the culture medium of the MBP-fusion proteins has not a cytotoxicity effect on cells in culture.

### Example 5. Evaluation of the MBP-fusion protein stability in cells

COS cells were treated with 50 µg/ml of recombinant MBP-melusin for 2 hours and then lysed immediately or after 24 hours or 4 days, or treated for 4 days and then lysed. Total cell extracts from the different samples and from untreated COS cells (NT) were subjected to western blot analysis using a anti-melusin antibody. MBP-melusin showed a high stability in all the sample tested (figure 4) demonstrating low degradation rate of this recombinant protein after cellular uptake.

### Example 6. Evaluation of MBP-fusion protein activity in cells

Primary rat cardiomyocytes untreated (NT) or treated for different times (10 seconds, 4, 24, 48, 96 hours) with 50 µg/ml of recombinant MBP-melusin were washed 10 times with 10 ml of PBS and extracted with lysis buffer (60 mM Tris pH 6.8, 1 % SDS). Since we previously demonstrated that melusin overexpression is able to activate ERKs pathway in cardiomyocytes, we evaluated ERK phosphorylation in treated and untreated cells. Total cell extracts were subjected to western blot analysis with anti-phospho ERK ½ and anti-total ERK1 polyclonal antibodies. As shown in figure 6 ERK1/2 phosphorylation was increased after 48 and 96 hours of treatment underlining MBP-melusin activity in cardiomyocytes.

Naturally, while the principle of the invention remains the same, the details of construction and the embodiments may widely vary with respect to what has been described and illustrated purely by way of example, without departing from the scope of the present invention as defined in the appended claims.

## Claims

1. System for delivering a molecule of interest cargo moiety into a cell, **characterized in that** said system comprises maltose binding protein as a transport moiety.

2. System according to claim 1, **characterized in that** the maltose binding protein is fused to the cargo moiety.

3. System according to claim 1, **characterized in that** the maltose binding protein is linked to the cargo moiety.

4. System according to claim 3, **characterized in that** the link is a chemical bond, preferably a covalent bond.

5. System according to claim 3, **characterized in that** the maltose binding protein is coupled to the cargo moiety .

6. System according to claim 5, **characterized in that** the coupling is achieved by means of the maltose-maltose binding protein complex, and the cargo moiety is linked to the maltose.

7. System according to any one of the preceding claims, **characterized in that** the molecule of interest cargo moiety is a protein, a nucleic acid molecule, an organic compound, a drug, a vaccine.

8. System according to any one of the preceding claims, **characterized in that** the maltose binding protein is produced via DNA recombinant technique.

9. System according to claim 9, **characterized in that** the recombinantly produced maltose binding protein is subsequently purified by affinity chromatography.

10. Method for delivering a molecule of interest cargo moiety into a cell, **characterized in that** the method comprises the steps of:
i) association of maltose binding protein with the molecule of interest cargo moiety ;
ii) administration of the association to the cell.

11. Method according to claim 10, **characterized in that** the association is achieved by means of i) fusion of the DNA coding sequences of maltose binding protein to the cargo moiety; ii) chemical conjugation of maltose binding protein to the cargo moiety; iii) coupling the maltose binding protein to the cargo moiety by means of the maltose-maltose binding protein complex, wherein the cargo moiety is chemically conjugated to the maltose.

12. Method according to claim 10, **characterized in that** the maltose binding protein is produced via DNA recombinant techniques.

13. Method according to claim 12, **characterized in that** the recombinantly produced maltose binding protein is subsequently purified by affinity chromatography.

14. Use of maltose binding protein as a transport moiety for the delivery of a molecule of interest cargo moiety into a cell.
